# EUROPEAN PATENT APPLICATION

(11) **EP 0 905 115 A1**
(43) Date of publication of application: **31.03.1999**
(21) Application number: 98870194.2
(22) Date of filing: 16.09.1998
(51) Int. Cl.: C07C 41/56, C07C 43/303, C11D 3/50

(54) **Method for making acetal compounds**

(30) Priority: 26.09.1997 US 17289
(71) Applicant: THE PROCTER & GAMBLE COMPANY, Cincinnati, Ohio 45202 (US)
(72) Inventor: Masami, Ono, Higashinada-ku, Kobe 658 (JP); Masahiro, Ishida, Nagata-ku, Kobe 653 (JP)
(74) Representative: Engisch, Gautier

(57) **Abstract**

A method for making an acetal compound is disclosed, comprising the steps of: (a) adding a carbonyl compound, an alcohol, and a dehydrating agent to an environment wherein the carbonyl compound is selected from the group consisting of an aldehyde, a ketone, and mixtures thereof; and (b) pressurizing the mixture of step (a), wherein the pressurization causes the carbonyl compound and the alcohol to react and form the acetal compound.

## Description

### FIELD

The present invention relates to a method for making acetal compounds.

### BACKGROUND

Acetal compounds are known in the art and have been widely used in many industries, for example, in the manufacture of pharmaceutical products and detergent products, and in the fragrance and lacquer industries. The term "acetal compound" is widely used to encompass both acetals derived from an aldehyde and an alcohol, and ketals derived from a ketone and an alcohol. Certain methods for making acetal compounds are also known in the art.

Conventional methods for making acetal compounds derive the acetal compound from an aldehyde and an alcohol with an acidic catalyst under reflux. The use of dehydrating agents (*e.g.*, molecular sieves) for removal of water during such a reaction is also known in the art. Removal of water obtained from such a dehydration reaction tends to shift the reaction equilibrium to the right, meaning that the reaction proceeds to derive the resulting acetal compounds from the starting compounds. Generally, Dean-Stark apparatus is used for carrying out the dehydration reaction in the formation of esters or acetals. See Synthesis, vol.7, pages 501-522, 1981, "Methods for the Preparation of Acetals from Alcohols or Oxiranes and Carbonyl Compounds," issued by Frans A.J. Meskens.

Certain alcohols such as low boiling point alcohols and unstable alcohols, however, may not be suitable for such known methods using the dehydration reaction (*e.g.*, acetal formation by Dean-Stark apparatus). Alcohols having low boiling points tend to volatilize during a reaction under reflux. The loss of the alcohol by such volatilization results in a much lower yield of the acetal compound as compared to the theoretical yield of acetal compound.

Herein, "unstable alcohol" means an alcohol which tends to decompose or degrade at high temperature in the acetal formation, especially in the existence of acidic catalysts under reflux. Generally, the harsher the condition (*e.g.*, high temperature, high pressure, etc.), the shorter the time required for the reaction, thereby the reaction can proceed more effectively, with high yield. However, if the reaction for the acetal formation using conventional methods (*e.g.*, by Dean-Stark apparatus) is carried out under harsh conditions, the use of unstable alcohols may cause undesirable chemical reaction between components, and/or decomposition or degradation of the starting components during the reaction under reflux, especially in the existence of an acidic catalysis.

In the use of such alcohols for acetal formation, two-step reactions may be considered instead of one-step reactions. The term "one-step" reaction means that the proposed compound is derived from starting components directly; in a "two-step" reaction, the proposed compound can not be directly derived. For example, a dialkyl acetal (*e.g.*, dimethyl acetal) wherein the alkyl unit has a short carbon chain length (*e.g.*, methyl) is obtained by the first step. The alkyl unit obtained is then substituted during the second step to obtain the proposed acetal. However, due to the need for multiple steps and much longer the reaction time, two-step reactions are generally less desirable than one-step reactions.

Consequently, the use of unstable alcohols in conventional methods for acetal formation typically result in undesirable low purity and yields of acetal compounds, or the acetal compounds may not be obtainable at all.

Based on the foregoing, there is a need for a method to obtain acetal compounds directly from carbonyl compounds and alcohols, including unstable alcohols, with high purity and yield of the acetal compound. There is also a need to facilitate the reaction, such as by reducing the number of reaction steps and the reaction time. None of the existing art provides all the benefits of the present invention.

### SUMMARY

The present invention is directed to a method for making an acetal compound comprising the steps of: (a) adding a carbonyl compound, an alcohol, and a dehydrating agent to a sealable environment wherein the carbonyl compound is selected from the group consisting of an aldehyde, a ketone, and mixtures thereof; and (b) pressurizing the mixture of step (a), wherein the pressurization causes the carbonyl compound and the alcohol to react and form the acetal compound.

These and other features, aspects, and advantages of the present invention will become better understood from a reading of the present disclosure.

### DETAILED DESCRIPTION

While the specification concludes with claims particularly pointing out and distinctly claiming the invention, it is believed that the present invention will be better understood from the following description.

The following is a list of definitions for terms used herein.

"Acetal compound" includes a compound derived from a starting carbonyl compound and an alcohol; acetals derived from aldehydes and alcohols; and ketals derived from ketones and alcohols.

"Carbonyl compound" means a compound having at least one carbonyl unit, including aldehydes, ketones, and carboxylic acids having -COOR unit; preferably, aldehydes or ketones.

"Alkyl" means a carbon-containing chain, which may be straight, branched, saturated, or cyclic; substituted (mono- or poly-) or unsubstituted.

"Alkenyl" means a carbon-containing chain, which may be straight, branched, unsaturated, or cyclic; substituted (mono- or poly-) or unsubstituted.

"Aryl" means an aromatic; substituted (mono- or poly-) or unsubstituted.

"Aralkyl" means arylalkyl which has the structure R'-R"-, wherein R' is an aryl and R" is an alkyl.

"Alkoxyl" means alkyloxy which has the structure R-O-.

"Polyalkoxylate" means -(R-O)ₙ-, wherein n is an integer.

"Amine" means R-NH₂.

"Amide" means R'-CONH₂ *or* R'-CONH-R", wherein R' and R" is independently alkyl or aryl.

"Ester" means R'-COO-R", wherein R' and R" is independently alkyl or aryl.

"Ether" means R'-O-R", wherein R' and R" is independently alkyl or aryl.

"Comprising" means that other steps and other components which do not affect the end result can be added. This term encompasses the terms "consisting of' and "consisting essentially of."

All percentages and ratios used herein are by weight of total composition, unless otherwise indicated.

All measurements referred to herein are made at 25°C unless otherwise specified.

All percentages, ratios, and levels of ingredients referred to herein are based on the actual amount of the ingredient, and do not include solvents, fillers, or other materials with which the ingredient may be combined as a commercially available product, unless otherwise indicated.

All publications, patent applications, and issued patents mentioned herein are hereby incorporated in their entirety by reference. Citation of any reference is not an admission regarding any determination as to its availability as prior art to the claimed invention.

The present invention relates to a method for making an acetal compound comprising the steps of: (a) adding a carbonyl compound, an alcohol, and a dehydrating agent to a sealable environment wherein the carbonyl compound is selected from the group consisting of an aldehyde, a ketone, and mixtures thereof; and (b) pressurizing the mixture of step (a), wherein the pressurization causes the carbonyl compound and the alcohol to react and form the acetal compound.

The method of the present invention can be used for the formation of acetal compounds wherein conventional methods (*e.g.*, using Dean-Stark apparatus) have been useful, but have typically required longer reaction times and/or typically have resulted in low purity and lower yields of acetal compounds. The acetal compounds derived from the method of the present invention can be used for a variety of the products in different industries, and are particularly useful as pro-fragrance acetals in detergent compositions, which are designed to readily disperse in aqueous solution, deposit on fabrics, and release perfume. Herein, "pro-fragrance" compound means a compound which may or may not be odoriferous in itself but which, upon hydrolysis, produces a desirable odor which is characteristic of one or more of its hydrolysis products.

### ACETAL COMPOUNDS

The acetal compounds of the present invention, including a acetal and a ketal, have following general formula:

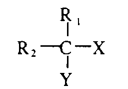

R₁ and R₂ are independently, alkyl, alkenyl, aryl, or aralkyl. X and Y are independently an alkoxy or polyalkoxylates. Preferably, the alkoxy has from 1 to about 30 carbons. The acetal compound of the present invention can be cyclic and acyclic. The term "cyclic and acyclic" in this context refers to the presence or absence of a covalent bond connecting moieties X and Y of the acetal compound. X and Y of the cyclic acetal compound form a ring structure.

R₁ and R₂ are derived from a starting carbonyl compound, and X and Y are derived from a starting alcohol, as described later.

A wide range of acetal compounds may be derived by the method of the invention, including those used in detergent compositions as pro-fragrance acetal compounds. Such pro-fragrance acetal compounds include pro-fragrance acetals and pro-fragrance ketals.

Specific preferred pro-fragrance acetals are nonlimitingly illustrated by the following: digeranyl citral acetal; didodecyl citral acetal; digeranyl vanillin acetal; didecyl α-hexyl cinnamic aldehyde acetal; didecyl cinnamic aldehyde acetal; didecyl citral acetal; didodecyl citral acetal; didecyl anis aldehyde acetal; diphenylethyl vanillin acetal; diphenylethyl ethyl vanillin acetal; digeranyl 2-methyl-3-(4-*t*-butylphenyl)-propanal acetal; didecyl dimethyl tetrahydrobenzaldehyde acetal; didodecyl dimethyl tetrahydrobenzaldehyde acetal; digeranyl decanal acetal; didodecyl decanal acetal; dicitronellyl hydroxycitronellal acetal; ditetradecyl hydroxycitronellal acetal; dioctadecyl 3-(3,4-methylenedioxyphenyl)-2-methylpropanal acetal; diphenylethyl citronellal acetal; di-3-methyl-5-phenyl pentanyl citronellal acetal; diphenylhexyl isocitral acetal; diphenylethyl 3-(*p*-ethylphenyl)-2,2-dimethyl-propanal acetal; di-2-ethylhexyl octanal acetal; di-9-decenyl 2-methyl-3-(4-*t*-butylphenyl)-propanal acetal; di-cis-3-hexenyl methyl nonyl acetaldehyde acetal; diphenylethyl 4-*t*-butyl-α-methylhydrocinnamic aldehyde acetal; dimethyl 2-methyl-3-(4-*t*-butylphenyl)-propanal acetal; and digeranyl 2-methyl-3-(4-isopropylphenyl)-propanal acetal.

Other acetals included in preferred embodiments of the present invention have the following general formula:

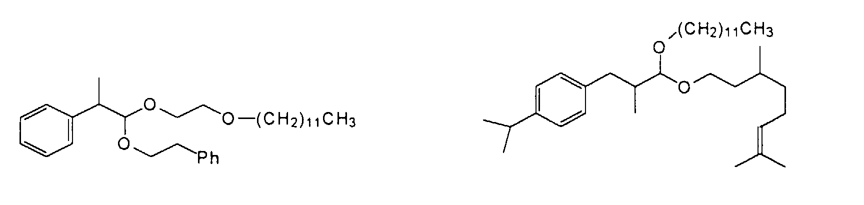

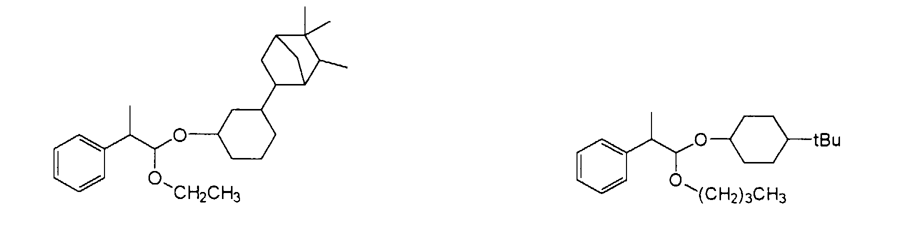

Other preferred acetals herein include: acetal 2-methyl-3-(4-*t*-butylphenyl)-propanal and ISOFOL or other branched alcohols from Condea Chemical; acetal of dimethyl tetrahydrobenzaldehyde and two moles of CH₃(CH₂)₁₁ OC(O)CH₂OH; acetal of 3-(*p*-ethylphenyl)-2,2-dimethyl-propanal and two moles of Neodol 1-3 detergent alcohol obtainable from Shell Chemical; diacetal of ethyl vanillin and pentaerythritol; acetal of lauryl aldehyde and two moles of 2-ethylhexanol.

Additional suitable acetals herein are cyclic acetals including the polyhydroxyamides. Typical examples of suitable polyhydroxyamides include the C₁₂-C₁₈ N-methylglucamides. See, for example, WO 9,206,154. Other sugar-derived acetal or ketal parent compounds useful herein include the N-alkoxy polyhydroxy fatty acid amides, such as C₁₀-C₁₈ N-(3-methoxypropyl) glucamide.

Specific preferred ketals are nonlimitingly illustrated by the following: diphenylethyl α-ionone ketal; didodecyl α-ionone ketal; diphenylhexyl β-ionone ketal; dicitronellyl γ-methyl ionone ketal; ditetradecyl γ-methyl ionone ketal; didecyl methyl β-naphthyl ketone ketal; dioctadecyl *cis*-jasmone ketal; digeranyl damascenone ketal; di-*cis*-3-hexenyl methyl dihydrojasmonate ketal; didodecyl methyl dihydrojasmonate ketal; didecyl benzyl acetone ketal; di-2-ethylhexyl methyl amyl ketone ketal; didodecyloxyethyl methyl amyl ketone ketal; dioctadecyl carvone ketal; and digeranyl geranyl acetone ketal.

Other specific ketals herein include: 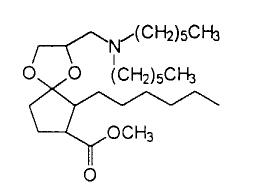 which is derived from methyl dihydrojasmonate and a dialkylaminopropanediol of the indicated chain-length (C₅); and 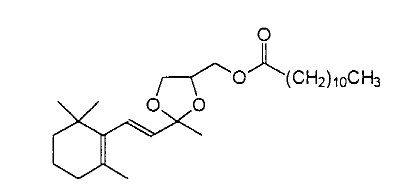 which is a ketal of β-ionone and a glyceryl fatty monoester having the indicated chain-length (C₁₀).

The method of the present invention further may be useful for making an ester and an imine compound wherein the same conventional methods for acetal formations (*e.g.*, using Dean-Stark apparatus) have been useful. The imine compound is derived from the carbonyl compound and a primary amine by hydrolysis.

When the method of the present invention is used to derive the imine compound, a primary amine is used instead of the alcohol in the reaction mixture. The carbonyl unit reacts with the primary amine by hydrolysis, thereby an imine having a schiff base (RCH=NR') is derived. Exemplary primary amines herein include any amine which is generally used in the formation of imine compounds.

When the method of the present invention is used to derive the ester compound, a carboxylic acid used as the carbonyl compound reacts with the alcohol.

### METHOD FOR MAKING ACETAL COMPOUND

### A. REACTION MIXTURE

The first step ("step (a)") of the present invention comprises adding a carbonyl compound, an alcohol, and a dehydrating agent to a sealable environment. The carbonyl compound, the alcohol, and the dehydrating agent can be added to the sealable enviroment without mixing, or can be mixed to obtain a more uniform mixture by any equipment which is generally used for mixing, shaking, or stirring ingredients and as long as a substantially airproof condition is obtained. In this step, a primary amine can be substituted for the alcohol for the formation of imine compound.

Herein, "sealable environment" means an environment which can be sealed by any conventional means such that it becomes substantially airproof, as preferred by a subsequent step in the process. The sealable environment can prevent the loss of alcohols which tends to volatilize during the reaction, especially those which are low boiling point alcohols. Further, due to the sealable environment, the alcohols which are once volatilized can be resolved in the solution, thereby there is no need to add excess alcohol to proceed acetal formation.

In addition, without being bound by theory, it is believed that when the environment is sealable, the pressure in the sealed environment tends to be higher due to the volatilization of certain alcohols, especially those which are low boiling point alcohols. The pressure increase can allow absorption of water produced during the reaction by dehydrating agents, resulting in proceeding the reaction to obtain the acetal compound.

Furthermore, a sealed environment may permit the use of lower temperatures for the reaction than those which are needed for the reaction using an unsealed environment. This means that the reaction carried out in a sealable environment may not require the harsher conditions (*e.g.*, higher temperatures and longer times) for proceeding the reaction speed or yield because undesirable decomposition or degradation of alcohols, especially those which are unstable, is avoided.

Preferred sealable environments include containers and chambers which may be readily heated at the next step. Preferably, the sealable environment is a tube or round type container.

### 1. Carbonyl compound

Preferably, the carbonyl compounds useful in the present invention are those having at least one carbonyl unit. The carbonyl compound may be in a solid or a liquid form. Preferably the carbonyl compound is selected from the group consisting of an aldehyde, a ketone, and mixtures thereof.

The aldehydes of the present invention useful herein include aliphatic, allylic, arylic, or benzylic. The aldehydes can be saturated, unsaturated, linear, branched, or cyclic, preferably C₉ or higher unsaturated aldehyde. The structures of the aldehydes can include alkyl, alkenyl, or aryl moieties. The aldehydes can further include those having additional functional groups such as alcohols, amines, amides, esters, or ethers as well.

Nonlimiting examples of the aldehydes include: hexyl aldehyde (caproaldehyde), heptyl aldehyde, octyl aldehyde (caprylaldehyde), nonyl aldehyde (pelargonaldehyde), decyl aldehyde (capraldehyde), undecyl aldehyde, dodecyl aldehyde (lauric aldehyde), tridecyl aldehyde, 3,5,5-trimethylhexanal, 2-methyldecanal (methyloctylacetaldehyde), 2-methylundecanal (methylnonylacetaldehyde), *trans*-2-hexenal (leaf aldehyde), *cis*-4-heptenal, *trans*-2-*cis*-6-nonadienal (cucumber aldehyde), *cis*-4-decenal, *trans*-4-decenal, 10-undecen-1-al (undecenoic aldehyde), *trans*-2-dodecenal, 2,6,10-trimethyl-9-undecenal, 2,6,10,-trimethyl-5,9-undecadienal, 3,7-dimethyl-2,6-octadienal (citral), 3,7-dimethyl-6-octen-1-al (citronellal), 7-hydroxy-3,7-dimethyloctan-1-al (hydroxy citronellal), *p*-mentha-1,8-dien-7-al (perilla aldehyde), 3,7-dimethyl-7-methoxyoctan-1-al, (methoxydihydrocitronellal), citronellyloxy acetaldehyde, 2,4-diemthyl-3-cyclohexenyl carboxyaldehyde, 2,4,6-trimethyl-3-cyclohexene-1-carboxyaldehyde (isocyclocitral), 5-methoxyoctahydro-4,7-menthano-1*H*-indene-2-carboxyaldehyde (scentenal), 4-(4-methyl-3-pentenyl)-3-cyclohexen-1-carboxyaldehyde (myrac aldehyde), 4-(4-hydroxy-4-methyl-pentyl)-3-cyclohexen-1-carboxyaldehyde (lyral), 1-methyl-4-(4-methylpentyl)-3-cyclohexen-carboxyaldehyde (vernaldehyde), 4-(tricyclo[5.2.1.0^{2,6}]decyliden-8)-butenal (dupical), 7-formyl-5-isopropyl-2-methyl-bicyclo-[2.2.2]oct-2-ene (maceal), 2-methyl-4-(2,6,6-trimethyl-1-cydohexen-1-yl)-2-butenal (boronal), 2-methyl-4-(2,6,6-trimethyl-2-cyclohexen-1-yl)-butanal (cetonal), benzaldehyde, phenylacetaldehyde (hyacinth aldehyde), 3-phenylpropanal (phenylpropylaldehyde), 3-pheny-2-propenal (cinnamic aldehyde), 2-pentyl-3-phenyl-2-propenal (α-amyl cinnamic aldehyde), 2-hexyl-3-phenyl-2-propenal (α-hexyl cinnamic aldehyde), 2-phenylpropanal (hydratropic aldehyde), 4-methoxybenzaldehyde (anis aldehyde), *p*-methylphenylacetaldehyde (*p*-tolyl acetaldehyde), 4-isopropylbenzaldehyde (cumin aldehyde), 2-methyl-3-(4-isopropylphenyl)-propanal (cyclamen aldehyde), 3-(*p*-*t*-butylphenyl)-propanal, 3-(*p*-ethylphenyl)-2,2-dimethylpropanal (*p*-ethyl 2,2-dimethylhydrocinnaldehyde), 2-methyl-3-(*p*-methoxyphenyl)-propanal, 2-methyl-3-(4-*t*-butylphenyl)-propanal (4-*t*-butyl-α-methylhydrocinnamic aldehyde, lily aldehyde), 2-hydroxybenzaldehyde (salicylic aldehyde), 3,4-methylenedioxy-benzaldehyde (heliotropine), 2-methyl-3-(3,4-methylenedioxy-phenyl)-propanal (helional), 4-hydroxy-3-methoxybenzaldehyde (vanillin), 3-ethoxy-4-hydroxybenzaldehyde (ethyl vanillin), 3,4-dimthyoxybenzaldehyde (methyl vanillin): aldehydes having low volatility by virtue of incorporation of bulky polar moieties.

The ketones useful herein include aliphatic, allylic or benzylic. The ketones can contain saturated, unsaturated, linear, branched, or cyclic, preferably including alkyl, alkenyl, or aryl moieties. The ketones can include other functional groups such as amides, amines, ethers, or esters.

Nonlimiting examples of the ketones include: 3-hydroxy-2-butanone (acetoine), 2,3-butanedione (diacetyl), 2-heptanone (methyl amyl ketone), 3-octanone (ethyl amyl ketone), 2-octanone (methyl hexyl ketone), 2-undecanone (methyl nonyl ketone), 6-methyl-5-hepten-2-one, acetyl diisoamylene (koavone), 1,7,7-trimethyl bicyclo[2.2.1]heptan-2-one(camphor), 1,8-*p*-menthadien-6-one (carvone), *p*-menthan-3-one (menthone), *d*-*p*-menth-4(8)-en-3-one (*d*-pulegone), *p*-menth-1-en-3-one (piperitone), 1,3,3-trimethyl-bicyclo[2.2.1]heptan-2-one (fenchone), 6,10,-dimethyl-5,9-undecadiene-2-one (geranyl acetone), acetyl cedrene (cedryl methyl ketone), 5,6-dimethyl-8-isopropenylbicyclo-[4.4.0]-1-decen-3-one (nootkatone), 4-(2,2,6-trimethyl-2-cyclohexen-1-yl)-3-buten-2-one (α-ionone), 4-(2,2,6-trimethyl-1-cyclohexen-1-yl)-3-buten-2-one (β-ionone), 5-(2,2,6-trimethyl-2-cyclohexen-1-yl)-4-pentan-3-one (α-methyl ionone), 5-(2,2,6-trimethyl-1-cyclohexen-1-yl)-4-pentan-3-one (β-methyl ionone), 5-(2,2,6-trimethyl-2-cyclohexen-1-yl)-3-methyl-3-buten-2-one (γ-methyl ionone), 5-(2,2,6-trimethyl-1-cyclohexen-1-yl)-3-methyl-3-buten-2-one (δ-methyl ionone), 1-(2,2,6-trimethyl-2-cyclohexen-1-yl)-1,6-heptadien-3-one (allyl ionone), α-irone, β-irone, γ-irone, 1-(2,2,6-trimethyl-2-cyclohexen-1-yl)-2-buten-1-one (α-damascone), 1-(2,2,6-trimethyl-1-cydohexen-1-yl)-2-buten-1-one (β-damascone), 1-(2,2,6-trimethyl-3-cyclohexen-1-yl)-2-buten-1-one (δ-damascone), 1-(3,3-dimethyl-6-cyclohexen-1-yl)-penta-4-en-1-one (α-dynascone), 1-(3,3-dimethyl-1-cyclohexen-1-yl)-penta-4-en-1-one (β-dynascone), 3-hydroxy-2-methyl-4H-pyran-4-one (maltol), 2-ethyl-3-hydroxy-4H-pyran-4-one (ethyl maltol), 2,5-diemthyl-4-hydroxy-2*H*-furan-3-one, 4,5-dimethyl-3-hydroxy-5*H*-furan-2-one (sugar lactone), *p*-*t*-butylcyclohexanone, 2-amylcyclopentanone, 2-heptylcyclopentanone, 3-methyl-2-pentyl-2-cyclopenten-1-one (dihydrojasmone), 3-methyl-2-(2-*cis*-penten-1-yl)-2-cyclopenten-1-one (*cis*-jasmone), 6 (or 7)-ethylidene-octahydro-5,8-methano-2*H*-benzopyrane (florex), 7-methyl-octahydro-1,4-methanonaphthalen-6(2*H*)-one (plicatone), 4-cyclohexyl-4-methyl-2-pentanone, 1-(*p*-menthen-6(2)-yl)-1-propanone, 2,2,5-trimethyl-5-pentylcyclopentanone, 4-(1-ethoxyvinyl)-3,3,5,5-tetramethyl-cyclohexanone, 6,7-dihydro-1,1,2,3,3-pentamethyl-4(5*H*)-indanone,7-acetyl-1,2,3,4,5,6,7,8-octahydro-1,1,6,7-tetramethylnaphthalene (Iso E Super), methyl 2,6,10-trimethyl-2,5,9,cyclododecatrien-1-yl ketone (trimofix "O"), acetophenone (methyl phenyl ketone), *p*-methyl acetophenone (*p*-tolyl methyl ketone), benzyl acetone, 7-methyl-3,4-dihydro-(2*H*)-1,5-benzodioxepin-3-one (calone), 4-(4-hydroxyphenyl)-2-butanone (raspberry ketone), *p*-methoxyphenylbutanone (anisyl acetone), 4-(4-hydroxy-3-methoxyphenyl)-2-butanone ("Zingerone"), 2-acetonaphthone (methyl β-naphthyl ketone), 4-phenyl-4-methyl-2-pentanone, and benzophenone (diphenyl ketone). Other exemplary ketones include Ethyl ketopropinate (ethyl pyruvate), isoamyl ketopropionate (isoamyl pyruvate), ethyl acetoacetate, ethyl γ-ketovalerate (ethyl levulinate), methyl jasmonate, and methyl dihydrojasmonate.

### 2. Alcohols

The alcohols useful in the present invention can be any alcohols which are commercially available and known in the art. The alcohols can be saturated, unsaturated, linear or branched compound, preferably those having alkyl, alkenyl, aryl, aralkyl, alkylalkoxylate derivatives with one or more OH groups. Preferably, the alcohol can be alkoxylated with 1 to 30 units of ethylene oxide, propylene oxide or mixtures thereof. Aromatic or aliphatic alcohols are useful. The alcohols can contain additional functionality such as amines, amides, ethers, or esters as a part of their structure. Low boiling point alcohols are particularly useful herein as a intermidiate for making other compound.

The alcohols useful herein include low boiling point alcohols and unstable alcohols and can be in a solid or liquid form. "Low boiling point alcohol" herein means an alcohol tending to volatilize under reflux during reaction with solvent using Dean-Stark apparatus. The low boiling point alcohols useful herein can include methyl alcohol and ethyl alcohol.

The unstable alcohols useful herein can include unsaturated alcohols or natural products. Nonlimiting examples of the unstable alcohol include, but are not limited to *trans*-2-hexenol, *cis*-3-hexenol, 1-octen-3-ol (amyl vinyl carbinol), 9-decenol, 4-methyl-3-decen-5-ol, 10-undecenol, and *trans*-2-*cis*-6-nonadien-1-ol (violet leaf alcohol).

Other exemplary alcohols include 3,7-dimethyl-1,6-octadiene-3-ol (linalool), 3,7-dimetyl-*trans*-2,6-octadien-1-ol (geraniol), 3,7-dimethyl-*cis*-2,6-octadien-1-ol (nerol), 3,7-dimethyl-6-octen-1-ol (citronellol), 2-methyl-6-methylene-7-octen-2-ol (myrcenol), 2,6-dimethyl-5-hydroxymethyl-2,6-heptadien (lavandulol), 3,7-dimethyloctanol (tetrahydrogeraniol), 3,7-dimethyloctan-3-ol (tetrahydrolinalool), 3,7-dimethyloctan-1,7-diol (hydroxy citronellol), 2,6-dimethyl-7-octen-2-ol (dihydromyrcenol), 3,7-dimethyl-4,6-octadien-3-ol (allo-ocimenol), *p*-menthan-8-ol (α-terpineol), 1-*p*-menthen-4-ol (terpinen-4-ol), *p*-menth-8-en-3-ol (isopulegol), 6,6-dimethyl-bicyclo-[3.1.1]-2-heptene-2-ethanol (nopol), 3,7,11-trimethyl-2,6,10-dodecatrien-12-ol (farnesol), 3,7,11-trimethyl-1,6,10-dodecatrien-3-ol (nerolidol), (-)-α-bisabolol (bisabolol), patchouli alcohol, vetiverol, 2,4-dimethyl-3-cyclohexene-1-methanol, 4-isopropylcyclohexanol, 2,5,5-trimethyl-1,2,3,4,4α,5,6,7-octahydro-2-naphthalenol (ambrinol), 4-methoxybenzyl alcohol (anis alcohol), 3-phenyl-2-propen-1-ol (cinnamic alcohol), 2-methoxy-4-allylphenol (eugenol), 2-methoxy-4-(1-propenyl)-phenol (isoeugenol), 5-propenyl-2-ethoxyphenol (propenyl guaethol), santalol, 2-ethyl-4-(2,2,3-trimethyl-3-cyclopenten-1-yl)-2-buten-1-ol (bacdanol), 2-methyl-4-(2,2,3-trimethyl-3-cyclopenten-1-yl)-2-buten-1-ol (sandalmysore core), and 3-methyl-5-(2,2,3-trimethyl-3-cyclopenten-1-yl)-4-penten-2-ol (ebanol).

### 3. Dehydrating Agent

"Dehydrating agent" herein means a component which absorbs and removes water formed during the reaction for proceeding acetal formation to obtain acetal compounds. The preferred dehydrating agent is molecular sieves, preferably a molecular sieve 3A, 4A, or 5A grade, which are commercially available.

The amount of the dehydrating agent added should be of a sufficient level for removal of water formed for substantially completing the reaction for making the acetal compound.

### 4. Additinal ingredients

The method of the present invention may further include additional ingredients, including catalysts and solvents into the sealable environment. The solvent is used for increasing vapor pressure and/or solvating those compounds which are in a solid form. Typically any or all of the aldehyde, the alcohol, the resulting acetal compound, and mixtures thereof may be in a solid form. The solvents useful in the present method can be any solvent used in the conventional dehydration reaction by Dean-Stark apparatus. Preferably, the solvent has sufficient stability during the pressurizing step, having a boiling point of at least 50°C, more preferably from about 50°C to about 80°C. "Sufficient stability" herein means a solvent which does not cause decomposition or degradation as a side-reaction during the heating step. The preferred solvent is hexane, benzene, or toluene.

The method of the present invention can generally proceed under a catalyst, preferably, a Lewis acid. Exemplary acidic catalysts are p-tolurene sulfonic acid, methane sulfonic acid, sulfuric acid, hydrochloric acid, sulfosalicylic acid, and mixtures thereof. Non-acidic catalysts are also useful in the present invention. Examples are Girder KSF, boron trifluoride etherate, potassium hydrogen sulfate, copper sulfate, and ion excharger.

### B. PRESSURIZING

A subsequent step ("step (b)") of the present invention comprises pressurizing the mixture of step (a), wherein the pressurization causes the carbonyl compound and the alcohol to react and form the acetal compound. Herein, "pressurizing" or "pressurization" means changing any condition of the reaction for deriving an acetal compound from the carbonyl compound and the alcohol, by increasing the temperature. Pressurizing herein includes the conditions produced by physically decreasing the volume by pressure, filling the environment with a gas stream which provides high pressure, and heating; preferably by heating.

The temperature and the time period used in the pressurizing of step (b) is selected depending upon the properties of the starting components, especially the boiling point of the alcohols, and the solvent if added. These conditions are further dependent upon the desired properties of the resulting compounds (such as crystal structure and chemical property).

The sealable environment can be heated to substantially complete the reaction. Preferably the temperature of heating is at least about 50°C, more preferably from about 50°C to about 80°C. Preferably the time and temperature of heating is from about 50°C to about 80°C for about overnight in an air-tight container.

The process of the present invention results in improved yields of the acetal; preferably at least a 90% yield. Herein, "yield" means the percentage of finished material obtained from the raw material. The concentration of the starting material can be determined by using NMR (Nuclear Magnetic resonance spectrum).

### EXAMPLES

The following examples further describe and demonstrate embodiments within the scope of the present invention. The examples are given solely for the purpose of illustration and are not to be construed as limitations of the present invention, as many variations thereof are possible without departing from the spirit and scope of the invention.

### Example 1

A 100 ml portion of benzene, 0.20 mol of 2-methyl-3-(4-*t*-butylphenyl)-propanal (lily aldehyde), 0.83 mol of methyl alcohol, 0.58 mmol of *p*-toluenesulfonic acid and 20 g of molecular sieves (3A) are added to a glass tube. The glass tube is sealed to be substantially air-tight and kept at 80°C overnight. The reaction mixture is washed several times with aqueous Na₂CO₃ solution and is extracted as an organic solution. The organic solution is then dried over Na₂SO₄. The organic solution is evaporated in vacuo after filtration. A 96.7% yield of dimethyl 2-methyl-3-(4-*t*-butylphenyl)-propanal acetal is obtained (conversion of aldehyde is 98% based on NMR). The purity of the resultant material is also determined by NMR.

### Example 2

A 50 ml portion of benzene, 41.7 mmol of 2-methyl-3-(4-isopropylphenyl)-propanal (cyclamen aldehyde), 167 mmol of geraniol, 0.32 mmol of p-toluenesulfonic acid and 10g of molecular sieves (3A) are added to glass tube. The method is same as that described in Example 1. A 92% yield of digeranyl 2-methyl-3-(4-isopropylphenyl)-propanal acetal is obtained (conversion of aldehyde is 98% based on NMR spectroscopy). The purity of the resultant material is also determined by NMR.

### Example 3

A 50 ml portion of benzene, 131 mmol of 2-methyl-3-(4-*t*-butylphenyl)-propanal (lily aldehyde), 308 mmol of geraniol, 0.581 mmol of p-toluenesulfonic acid, and 30 g of molecular sieves (3A) are added to glass tube. The method is same as that described in Example 1. A 99% yield of di-geranyl 2-methyl-3-(4-*t*-butylphenyl)-propanal acetal is obtained (conversion of aldehyde is 98% based on NMR spectroscopy). The purity of the resultant material is also determined by NMR.

### Example 4

A 170 ml portion of benzene, 48.2 mmol of 2-methyl-3-(4-*t*-butylphenyl)-propanal (lily aldehyde), 145 mmol of 4-methyl cyclohexanol, 0.406 mmol of p-toluenesulfonic acid, and 10 g of molecular sieves (3A) are added to glass tube. The method is same as that described in Example 1. A 99% yield of di-geranyl 2-methyl-3-(4-*t*-butylphenyl)-propanal acetal is obtained (conversion of aldehyde was 98% based on NMR spectroscopy). The purity of the resultant material is also determined by NMR.

The embodiments disclosed and represented by the previous examples have many advantages. For example, the resulting acetal compounds derived by heating in the sealed container are generally of higher yield and higher purity than those obtained by known methods. In addition, acetal compounds are obtained with high yield and purity by the method of the present invention where they may not obtainable by conventional methods under substantially identical conditions, or where conventional methods require much longer reaction time. Due to the sealed container, the reaction can be facilitated, for example, obtaining the proposed compounds directly by the reaction, so that the reaction time is shorter than those of the known method.

Although the examples and embodiments described herein are illustrative of the invention, those skilled in the art will be able to recognize that variations or modifications in light thereof are fully within the scope of the invention.

## Claims

1. A method for making an acetal compound comprising the steps of:
(a) adding a carbonyl compound, an alcohol, and a dehydrating agent to a sealed environment wherein the carbonyl compound is selected from the group consisting of an aldehyde, a ketone, and mixtures thereof; and
(b) pressurizing the mixture of step (a), wherein the pressurization causes the carbonyl compound and the alcohol to react and form the acetal compound.

2. The method of Claim 1, wherein the pressurization is achieved by heating.

3. The method of Claim 2, wherein heating is at a temperature of at least about 50°C.

4. The method of Claim 3, wherein the alcohol is selected from the group consisting of a low boiling point alcohol and an unstable alcohol.

5. The method of Claim 4, wherein the low boiling point alcohol is selected from the group consisting of methyl alcohol and ethyl alcohol.

6. The method of Claim 5, wherein step (a) further comprises adding a solvent.

7. The method of Claim 6, wherein the reaction yields at least about 90% of the acetal compound.

8. The method of Claim 3, wherein the dehydrating agent is molecular sieves.

9. A acetal compound made by the method of Claim 1.

10. A detergent composition comprising the acetal compound made by the method of Claim 1.
